# EUROPEAN PATENT APPLICATION

(11) **EP 1 230 842 A1**
(43) Date of publication of application: **14.08.2002**
(21) Application number: 01102957.6
(22) Date of filing: 08.02.2001
(51) Int. Cl.: A01H 4/00, A01H 3/00

(54) **Campanula propagation**

(71) Applicant: GARTNERIET PKM ApS, 5270 Odense N (DK)
(72) Inventor: Jensen, Gert Kim, 5270 Odense N (DK)
(74) Representative: Roerboel, Leif

(57) **Abstract**

This invention pertains to a method of industrial propagation of rosette-forming Campanula. By treating a parent plant (1) with a high dose of a plant hormone belonging to the gibberellins, growth of buds (2) from growing points (3) is induced. The resulting shoots (4) are taken as cuttings which are planted in a growing substrate to grow a new plant like the one it came from.

## Description

### TECHNICAL FIELD

The present invention relates to an industrial method of propagating rosette-forming Campanula as set forth in the preamble of claim 1.

### BACKGROUND ART

Rosette-forming Campanula are normally reproduced on an industrial scale from seed. Propagation through cutting or rooting is possible for some species of rosette-forming Campanula in laboratory conditions through a cumbersome and problematic process that is not commercially viable for industrial propagation. There are several disadvantages connected to propagating through seeds. The market often demands Campanula having a particular appearance such as colour and shape of the flowers. When propagating through seeds, the resulting plants comprise a great variety in appearance because the genetic material is not identical. The consequence being that often more than half of the seeded plants cannot be sold and have to be destroyed. Further, the variation of properties causes the speed at which the plants develop to vary significantly, and the plants will flower after one, two or three years. The plants have to be flowering when sold. A span of years between the time of flowering of plants that were sown in the same batch is not complement with industrial propagation in greenhouses, where the efficiency of the space and time is crucial because of the high operating costs of a greenhouse. Thus, there is a need for an industrially viable method of propagating rosette-forming Campanula with uniform genetic material.

### DISCLOSURE OF THE INVENTION

On this background, it is the object of the present invention to provide a method of propagating rosette-forming Campanula of the kind referred to initially, which overcomes the above-mentioned problems. This object is achieved in accordance with claim 1. The treatment of the parent plants with a high dose of a plant hormone belonging to the gibberellins initiates the growth of buds at the grow points which grow into shoots that are suitable to be used as cuttings for propagation with a high rate of success.

According to an embodiment of the invention, the parent plants are treated with a gibberellin suspension with a concentration of 100 to 2000 ppm. Tests proved that concentration above this range does not improve the results, whereas a lower dose leads to reduced success rates.

According to another embodiment, the first generation of parent plants is reproduced from a selected plant by meristem cloning. Usually only a single plant with the required characteristics can be found. One parent plant does not provide sufficient cuttings to form the basis for large-scale propagation. As the trends in colour and shape change fast, it is not practical to slowly build-up a group of parent plants by taking cuttings. By using meristem cloning a sufficiently large stock of parent plants with ideal properties can be created fast enough to respond to market changes.

According to a further embodiment, the cuttings are treated with a plant hormone belonging to the auxins, to stimulate rooting in order to improve the success rate of the planted cuttings, thereby increasing the overall efficiency of the propagation method.

It is another object of the present invention to provide a method of controlling the growth and time of flowering of asexually reproduced rosette-forming Campanula, as defined in claim 8.

### BRIEF DESCRIPTION OF THE DRAWINGS

In the following detailed portion of the present description, the invention will be explained in more detail with reference to the exemplary embodiments shown in the drawings, in which
Figure 1 shows a rosette-forming Campanula,
Figure 2 shows a rosette-forming Campanula shortly after treatment with gibberellin, and
Figure 3 shows a rosette-forming Campanula having shoots from the growth points suitable for use as cuttings.

### DETAILED DESCRIPTION OF THE INVENTION

The genus Campanula comprises about 300 species of annuals, biennials and perennials. Most of these species are easily cultivated and provide a display of long lasting flowers.

Leaves are entire or toothed and borne alternately. Flowers are borne in panicles, racemes or clustered heads. Occasionally, they are solitary. Their shape varies from tubular to bell- or star-shaped. They may also be more open and be cup- or saucer-shaped.

Propagation for all but double-flowered forms is easy from seed, although some are slow to germinate and develop (e.g. Campanula Formanekiana). Seed may be sown in containers and placed in a cold frame in the spring.

The present invention pertains to the group of rosette-forming Campanula, i.e. forming a cluster of leaves growing in a circular overlapping pattern. This group, botanically also referred to as Rupestris/Lyrata group, comprises e.g. Campanula Formanekiana, Campanula Ephesia, Campanula Celsii, Campanula Lyrata and Campanula Incurva.

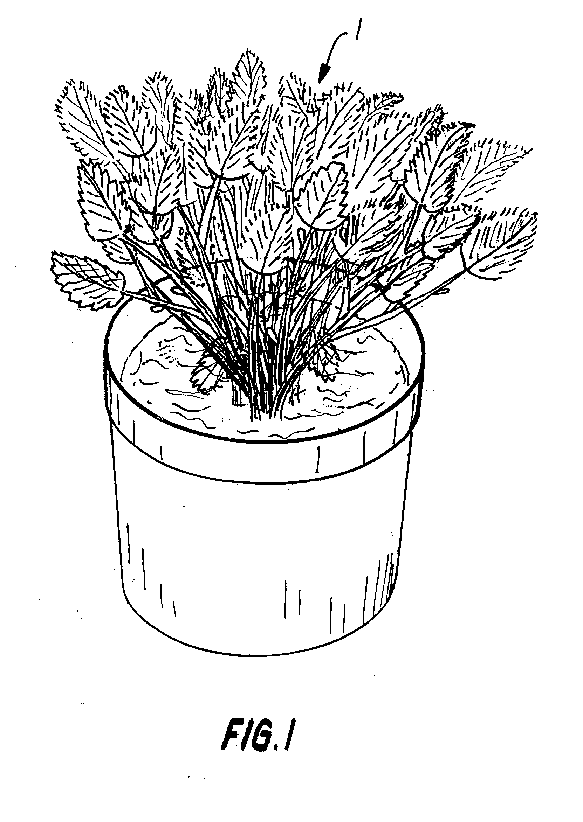

All species of the rosette-forming group are problematic to propagate by cuttings. Figure 1 shows a Campanula Formanekiana. None of the parts of the plant are, as far as known, suitable for taking viable cuttings.

According to a preferred embodiment of the invention, a high dose of a plant hormone belonging to the gibberellins is applied to a sufficiently developed parent plant 1. Commercially available gibberellins like gibberellic acid (GA3) are sold under the trademark Berelex, produced by Abbott Laboratories.

The gibberellic acid is applied by spraying a solution on the plants 1. Generally the concentration has to be between 100 and 2000 ppm. Tests showed that the preferred concentration of the gibberellic acid solution is 500 to 1000 ppm to apply a high dose. Below 500 ppm the viability of the cuttings and the number of cuttings are insufficient. Above 1000 ppm the tests showed no further improvement in the viability and number of cuttings.

It has shown to be advantageous to have a repetitive treatment under certain circumstances.

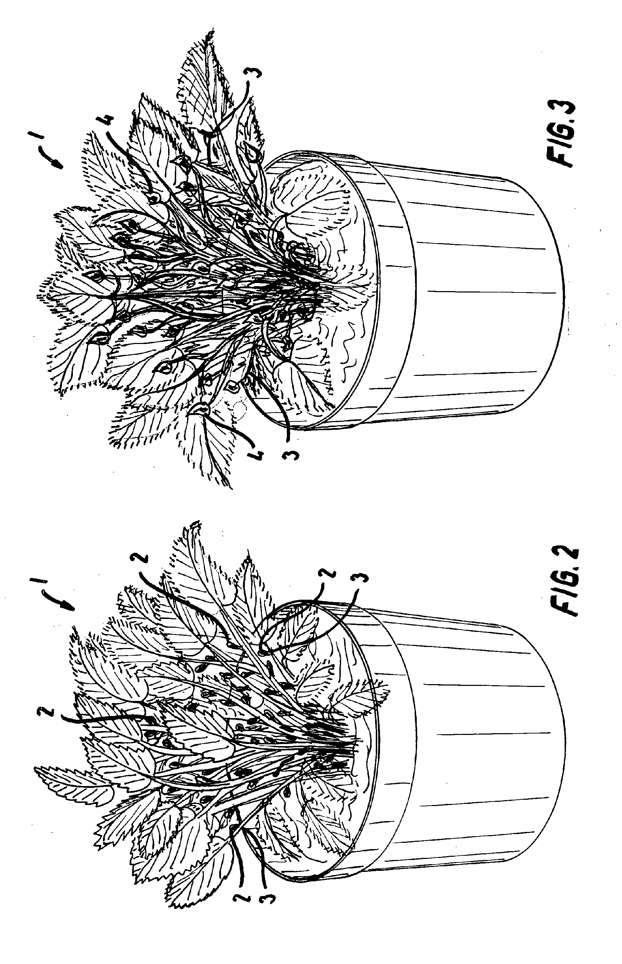

Figure 2 shows a plant 1 shortly after being treated with a high dose of gibberellin. The gibberellin treatment has initiated the growth of high numbers (approximately 10 buds per parent plant) of buds 2 from the growing points 3.

Figure 3 shows the plant in the next stage where the buds 2 have developed to shoots 4 that are of a suitable size for taking cuttings. These cuttings are planted in a suitable substrate (not shown) in order to grow a plant identical to the parent plant 1. In a preferred embodiment, the cuttings are treated with a plant hormone belonging to the auxins for stimulating root growth on the cuttings.

According to yet another preferred embodiment, the first generation of parent plants 1 is obtained by meristem cloning from a plant with particularly advantageous characteristics. In order to determine if a potential parent plant has particularly advantageous characteristics, it is necessary to let the plant flower so that its characteristics are displayed. The rosette-forming Campanula die, however, after flowering and are thus no longer suitable for use as parent plant. This dilemma is overcome by meristem cloning. From the meristem clone a vegetative plant with identical genetic material can be obtained which is suitable for use as a parent plant. Thus, a sufficient number of parent plants for taking cuttings can be produced relatively fast in order,to respond quickly to changes in trends of colour, shape and other characteristics dictated by the market.

Generally asexually propagated Campanula have problems with flowering because the natural mechanism for initiating generative growth has been put out of action. Thus, it has been proven experimentally that a winter simulation method as defined below stimulates generative growth:
- providing 10 to 12 weeks of long-day light conditions after planting, thereafter
- providing 2 to 4 weeks of short-day light conditions, then
- providing 3 to 6 weeks of substantially complete darkness and temperatures between 0 to 8° C, to simulate a winter, thereafter
- providing long-day light conditions to stimulate generative growth.

It has been proven to be advantageous when the plants are kept on low nutrients levels, e.g. 1.0, and on low humidity of the soil, i.e. the substrate must be allowed to dry out between the waterings to further stimulate generative growth.

In a preferred embodiment, the parent plants and the cuttings are cultivated in a greenhouse.

### LIST OF REFERENCE NUMERALS

- 1: plant
- 2: bud
- 3: grow point
- 4: shoot

## Claims

1. Method of asexually propagating rosette-forming Campanula comprising the steps of:
- applying a plant hormone belonging to the gibberellins to a parent plant (1), thereby inducing growth of buds (2) from growing points (3),
- allowing said buds (2) to grow to form shoots (4),
- taking said shoots (4) as cuttings, and
- planting said cuttings in a growing substrate to grow a new plant like the one it came from.

2. Method according to claim 1, wherein the gibberellin is applied as a solution with a concentration of 500 to 1000 ppm.

3. Method according to claim 1 or 2, wherein said solution is applied by spraying.

4. Method according to any of claims 1 to 3, wherein a parent plant is reproduced from a selected plant by meristem cloning.

5. Method according to any of claims 1 to 4 in which the cuttings are treated with auxin to stimulate rooting.

6. Method according to any of claims 2 to 5 in which the gibberelin solution is applied as a spray and/or as a soil drench.

7. Method according to any of claims 1-6 in which 0.0005 g to 0.020 g, preferably 0.003 g to 0.004 g, of giberellin is applied per plant.

8. Method of controlling the growth and time of flowering of rosette-forming Campanula asexually propagated
- providing 10 to 12 weeks of long-day light conditions after planting, thereafter
- providing 2 to 4 weeks of short-day light conditions, then
- providing 3 to 6 weeks of substantially complete darkness and temperatures between 0 to 8° C to simulate a winter, thereafter
- providing long-day light conditions to stimulate generative growth.

9. Method according to claim 8 wherein the plants are kept on low nutrients levels, and on low humidity of the soil.
